# EUROPEAN PATENT SPECIFICATION

(11) **EP 1 755 704 B1**
(45) Date of publication and mention of the grant of the patent: **27.02.2008**
(21) Application number: 05779816.7
(22) Date of filing: 27.05.2005
(51) Int. Cl.: A61M 5/168, A61M 5/00, A61M 31/00, A61M 5/178, B65B 3/00, A61J 3/00

(54) **SYSTEM, METHOD, AND COMPUTER PROGRAM PRODUCT FOR HANDLING, MIXING, DISPENSING, AND INJECTING RADIOPHARMACEUTICAL AGENTS**
SYSTEM, VERFAHREN UND COMPUTERPROGRAMMPRODUKT ZUR HANDHABUNG, MISCHUNG, AUSGABE UND INJEKTION RADIOPHARMAZEUTISCHER MITTEL
SYSTEME, PROCEDE ET PROGRAMME INFORMATIQUE POUR LA MANIPULATION, LE MELANGE, LA DISTRIBUTION ET L'INJECTION DE PRODUITS RADIOPHARMACEUTIQUES

(30) Priority: 27.05.2004 US 574875 P
(43) Date of publication of application: 28.02.2007
(73) Proprietor: E-Z-EM, Inc., Lake Success, NY 10042 (US)
(72) Inventor: WILLIAMS, Robert, C., Jr., Fort Salonga, NY 11678 (US)
(74) Representative: Price, Nigel John King
(86) International application number: PCT/US2005/018820
(87) International publication number: WO 2005/118031

(56) References cited:
- US-A- 5 569 181
- US-A- 5 592 940
- US-A- 5 911 252
- US-A1- 2001 034 506
- US-A1- 2001 049 608
- US-B1- 6 626 862
- PATENT ABSTRACTS OF JAPAN vol. 2000, no. 15, 6 April 2001 (2001-04-06) & JP 2000 350783 A (SUMITOMO HEAVY IND LTD), 19 December 2000 (2000-12-19)

## Description

### FIELD OF THE INVENTION

The present invention relates generally to handling, mixing, and/or dispensing systems and methods. More particularly the present invention relates to a system, method, and/or computer program product for the handling, mixing, dispensing, and/or injection of a mixture containing, for example, radiopharmaceutical agents, for use in various types of diagnostic imaging and/or therapeutic procedures.

### BACKGROUND OF THE INVENTION

Emissions from radioactive sources are often used in the medical field to provide imagery of internal body structures including, but not limited to, bone, vascular, organ systems, and other tissue. In addition, such emissions may also be used as therapeutic agents to inhibit the growth of targeted cells or tissue, such as, for instance, cancer cells. In addition, some pharmaceutical agents and/or radiopharmaceutical agents having hazardous physical and/or chemical effects when exposed to individuals (including, but not limited to, clinicians, imaging technicians, and pharmacists) are also often used in the medical field in therapeutic, diagnostic, and/or other medical procedures.

According to conventional radiographic diagnostic imaging techniques, such as X-ray procedures, X-rays pass through a target object and expose an underlying photographic film. The developed film then provides an image of the radiodensity pattern of the object. Less radiodense areas produce a greater blackening of the film; more radiodense, bony tissues produce a lighter image. Effective contrast agents for X-ray may be either less radiodense than body tissues or more radiodense. The less radiodense agents include, for example, air and other gases; an example of a more radiodense contrast material is a barium sulfate suspension.

Computed tomography (CT) is superior to conventional radiography in its ability to image, with extremely high resolution, a succession of thin sections of an object at specific points, lines or planes along the X, Y, or Z axis of the target object. However, because this procedure is also based on the detection of differences in radiodensity, requirements for contrast agents in CT are essentially identical with those for conventional radiography.

Magnetic resonance imaging (MR) systems for body imaging operate on a different physical principle. Generally, MR relies on the atomic properties (nuclear resonance) of protons in tissues when they are scanned with radio frequency radiation. The protons in the tissue, which resonate at slightly different frequencies, produce a signal that a computer uses to tell one tissue from another. MR can provide detailed three- dimensional soft tissue images.

Other imaging methods, however, that are used to obtain information about function-related tissues, may use radiopharmaceutical agents and/or other pharmaceutical agents as tracers to interact with the targeted tissues. These methods include, but are not limited to, procedures such as single photon emission computerized tomography (SPECT) and positron emission tomography (PET). SPECT uses a molecule normally found in the body in which one of the atoms of the molecule is replaced by a radioactive atom contained within a radiopharmaceutical agent that is injected into the individual. The radiopharmaceutical agent, which is chosen for its ability to interact with specific tissues, is sometimes called a tracer. The tracer emits photons that can be detected as the tissue is scanned at various angles or as the photons pass through a detector array. In certain embodiments, a computer reconstructs a 3-dimensional color tracer image. PET uses radiopharmaceutical agents as tracers to produce 3-D color images with a greater sensitivity than with SPECT. PET can be used in combination with CT to create a complimentary imaging effect in an imaging technique called CT-PET.

The radioactivity levels of the radiopharmaceutical agents used as tracers in, for instance, SPECT and PET procedures, are measured by medical personnel such as radio-pharmacists, to determine the radiation dose that the individual will receive during the course of a diagnostic procedure. The radiation dose received depends on a number of factors, including the half-life of the radiopharmaceutical agent (which, in turn, determines the total time the individual is exposed to radiation from the radiopharmaceutical agent), and the initial radioactivity level of the radiopharmaceutical agent at the time it is injected into the individual.

In PET imaging, an injectable radiopharmaceutical agent such as, for instance, FDG (fluorodeoxyglucose), is fabricated in a cyclotron device. Thereafter, the FDG may be transferred in a container device that may further comprise, for instance, an inner container device and a shielding, to prevent unnecessary radiation exposure to personnel, such as the radio-pharmacist, responsible for transporting/handling the FDG from the cyclotron to the PET imaging site. Since the half-life of FDG is short, approximately 110 minutes, it is necessary to quickly transport the FDG to the PET imaging site. Depending upon the elapsed transport time and the initial radioactivity level of the FDG at the time of fabrication, it is often required that the radioactivity level needs to be re-measured at the PET imaging site. Should a specific initial FDG radioactivity level, typically expressed milliCuries/milliliter be required at the time of patient injection, a radio-pharmacist at the PET imaging site may dilute the raw FDG with a diluent such as, for instance, IV saline solution, prior to loading the injection device with a specified volume. During this process, the handling of the FDG from container device to injection device for patient injection may be entirely manual. Within this process, several products are currently marketed to aid in shielding individuals from FDG during handling and dose calibration (measuring radiation). Although shielding may reduce the radiation exposure of the radio-pharmacist in handling the shielded vial, the radio-pharmacist may still be exposed to emissions from the radiopharmaceutical agent during the manual mixing and/or dilution process required to obtain the required dose. In addition, in some medical procedures, pharmaceutical agents or other materials emanating toxic and/or otherwise harmful emissions may be suitable for dispensing into an individual for diagnostic, therapeutic, and/or other medical procedures. It may be preferable, however, to shield individuals administering such procedures (including, but not limited to clinicians, pharmacists, and technicians), from the harmful emanations of such agents and/or materials.

Thus, there exists a need for a system, method, and/or computer program product for handling, mixing, dispensing, and injecting a mixture containing a first material, such as, for instance, a radiopharmaceutical agent and a second material, such as, for instance, an intravenous saline solution, such that an operator of such a system (e.g., a radio-pharmacist, clinician, or other individual) is subjected to reduced exposure to and/or reduced handling of the first material or mixtures formed that may contain the first material. In addition, there exists a need for a system and method for automatically mixing, diluting, and/or dispensing into an injection device such that the mixture containing, for instance, a radiopharmaceutical agent, provides a selected radiation dose amount when injected into an individual.

### SUMMARY OF THE INVENTION

In at least one alternative embodiment, the present invention provides a system comprising one or more container devices for holding a first set of one or more materials and one or more dispensing devices for holding a mixture of at least a portion of the first material and a set of one or more second materials, and other substances as well. The system of the present invention may also comprise one or more mixing devices or automated injector devices for receiving the containers so as to reduce the handling of the first material contained in the containers, for example. The mixing device may be further capable of mixing at least a portion of the first material with at least a portion of the second material according to a predetermined ratio to form a mixture. The mixing device may also direct the mixture to the dispensing device for dispensing one or more mixtures into an individual. In some embodiments of the present invention, the mixing device may be integrated with an automated injector device such that the power injection device may receive the one or more container devices to mix at least a portion of the first material with at least a portion of the second material according to a predetermined ratio to form the one or more mixtures that may then be automatically injected into an individual.

According to other aspects of the present invention, the present invention may further comprise one or more second containers for holding the second material, and a computer device operably engaged with the mixing device and configured to cooperate with the mixing device to produce the mixture according to the predetermined ratio. In other embodiments, the one or more container devices and the one or more dispensing devices may each further comprise one or more shieldings for shielding operators from radiation or other caustic or hazardous emissions that may emanate from the first or second materials and the resulting mixtures, respectively.

Another embodiment of the present invention comprises a method and/or computer program product for forming a mixture. Such a method may comprise steps for receiving at least a first container suitable for holding a first set of one or more materials at at least one mixing device, wherein the mixing device is capable of receiving the first container so as to reduce the manual handling of the first material that may be contained therein. At least a portion of the first material is mixed with at least a portion of a second material according to a predetermined ratio to form the mixture using the mixing device, the mixing device being further suitable for receiving one or more dispensing devices suitable for holding the mixture. The mixture formed by the mixing device is then directed to the one or more dispensing devices, wherein the one or more dispensing devices are suitable for dispensing the mixture so as to reduce the handling of the mixture contained within the dispensing device.

Embodiments of the present invention may also include a system and method whereby a mixture comprising at least a portion of a first and a second material is formed and transferred to a dispensing device wherein an operator using the system is minimally exposed to the first material and/or mixture formed by the mixing device of the system. Some embodiments of the present invention also provide a system wherein a computer device, in communication with a mixing device, may form a mixture by mixing a first material comprising, for instance, a radiopharmaceutical agent, and a second material, serving as a diluent, wherein the mixture is automatically formed having a predetermined radiation dose amount that is based on a predetermined ratio of the first material to the second material in the mixture.

Furthermore, according to other embodiments of the present inventions a method for preparing a radiopharmaceutical agent for injection into an individual is provided, which first comprises engaging one or more containers suitable for holding one or more materials, the one or more materials comprising one or more radiopharmaceutical agents, with a mixing device, so as to supply the materials to the mixing device and reduce handling of the materials contained in the one or more containers. The embodiment for preparing a radiopharmaceutical agent for injection further comprises the steps of: mixing, with the mixing device, the one or more materials according to a predetermined ratio to form the mixture; and directing the mixture to a dispensing device, the dispensing device being adapted for dispensing the mixture into an individual.

Such embodiments provide significant advantages as described and otherwise discussed herein.

### BRIEF DESCRIPTION OF THE DRAWINGS

Reference will now be made to the accompanying drawings, which are not necessarily drawn to scale. The drawings are for illustrative purposes only, and are not intended to limit the scope of the present invention.

**FIG. 1** shows a non-limiting schematic of a system for forming a mixture, including a container device, mixing device, and dispensing device, according to one embodiment of the present invention.

**FIG. 2** shows a non-limiting schematic of the present system for forming a mixture, according to one embodiment of the present invention showing the dispensing device configured as an injection device compatible with, for instance, a power injector.

**FIG. 3** shows a non-limiting schematic of the present system for forming a mixture including a mixing device substantially integrated with an automated injector device.

**FIG. 4** shows a non-limiting flow diagram according to the present method and computer program product for mixing a first material with a second material to form a mixture according to one embodiment of the present invention.

**FIG. 5** shows a non-limiting flow diagram according to the present method and computer program product for mixing a first material with a second material to form a mixture according to one embodiment of the present invention, including the step of dispensing the mixture to an individual.

**FIG. 6** shows a non-limiting flow diagram according to a method and computer program product for mixing multiple substances to form a mixture according to one embodiment of the present invention, including the steps of receiving dosage information and determining a predetermined ratio of each substance relative to each other in the mixture based on the dosage information.

**FIG. 7** shows a non-limiting flow diagram according to a method and computer program product for mixing a first material with a second material to form a mixture according to one embodiment of the present invention, including the steps of determining the radioactivity level of the first material and the mixture.

**FIG. 8** shows a non-limiting flow diagram according to a method and computer program product according to one embodiment of the present invention, wherein the method and computer program product are suitable for mixing a radiopharmaceutical device with a diluent such as, for example, IV saline solution, according to a predetermined ratio.

### DETAILED DESCRIPTION OF THE INVENTION

The present invention will be described with reference to the accompanying drawings, where applicable. It is understood that the present invention may be embodied in many different forms and should not be construed as limited to the embodiments set forth herein; rather, these embodiments are provided for illustrative purposes only. Like numbers refer to like elements throughout.

While the embodiments of the system and method for handling, mixing, dispensing and/or injecting mixtures including, for instance, radiopharmaceutical agents, are described below in the context of PET imaging techniques utilizing FDG as the first material, it should be understood that the embodiments of the present invention may also be utilized to handle, mix, dispense, and/or inject a variety of substances such that the substances are diluted, mixed, and/or manipulated such that the first and second materials are mixed according to a predetermined ratio to produce a mixture characterized by, for instance, a selected radiation dose amount. Further, the mixtures formed by the various embodiments of the present invention may be utilized in a variety of different imaging and/or therapeutic procedures requiring a mixture comprising a, pharmaceutical agent, for example.

As used herein, the term "material" may include, but it not limited to, one or more components which may include, but are not limited to, pharmaceutical agents, radiopharmaceutical agents, therapeutic agents, diagnostic agents, chemical compounds, diluents, flushing media, contrast media, or other materials that may be suitable and/or necessary for use in therapeutic, diagnostic, and/or medical procedures.

As used herein, the term "mixture" includes, but is not limited to, a combination, fusion, and/or blend of one or more of the materials or components described herein. According to some embodiments of the present invention, the mixture may be formed by physically and/or chemically mixing one or more of the materials or components described herein. Thus, "mixtures" of the present invention may include, but are not limited to, physical or chemical combinations of materials or components.

According to some embodiments, "radiopharmaceutical agents" may comprise radioactive materials capable of emitting radiation that may be harmful to individuals administering such material if the material is not shielded to reduce the amount of emitted radiation. Further, in some embodiments, pharmaceutical agents may also comprise toxic, caustic, and/or otherwise hazardous compounds such that the handling of such pharmaceutical agents by individuals should be reduced whenever possible.

The term "radiopharmaceutical agent" also includes, but is not limited to, a material, mixture, and/or pharmaceutical agent emitting radiation therefrom and/or providing a quantifiable radiation dose to an individual exposed thereto. Radiopharmaceutical agents may be capable of emitting radiation for treating a medical condition (for example, such as a cancerous tumor), diagnosing a medical condition (for example, by providing a radioactive marker that is detectable by an imaging or other diagnostic device), or providing images of a patient as part of a medical imaging procedure (for example, by acting as a radioactive marker or contrast media). The radiopharmaceutical agents of the present invention may be administered to an individual via injection, ingestion, or other suitable means. The term "radiation dose" includes, but is not limited to, an amount of radiation absorbed by an individual during a therapeutic, diagnostic, and/or medical procedure, wherein the radiation does may be measured using units of measure that may include, but are not limited to rem, Roentgen, curies, and/or other suitable units of measure for radiation dosage. According to embodiments of the present invention, a predetermined radiation dose may be calculated by a computer device with input from an individual in order to prepare a radiopharmaceutical agent capable of providing the predetermined radiation dose.

As used herein, the term "diluent" includes, but is not limited to, a material that may comprise liquid, solid, or gaseous materials suitable for dilution of one or more pharmaceutical agents, mixtures, or materials. Diluents may comprise various materials including, but not limited to, water, saline solutions, flushing media, intravenous solutions, or other materials suitable for diluting, flushing, or diminishing the effects of a pharmaceutical agent. In some embodiments, the diluent may be physically and/or chemically mixed with a radiopharmaceutical agent to prepare a mixture capable of providing a predetermined radiation dose when administered to an individual.

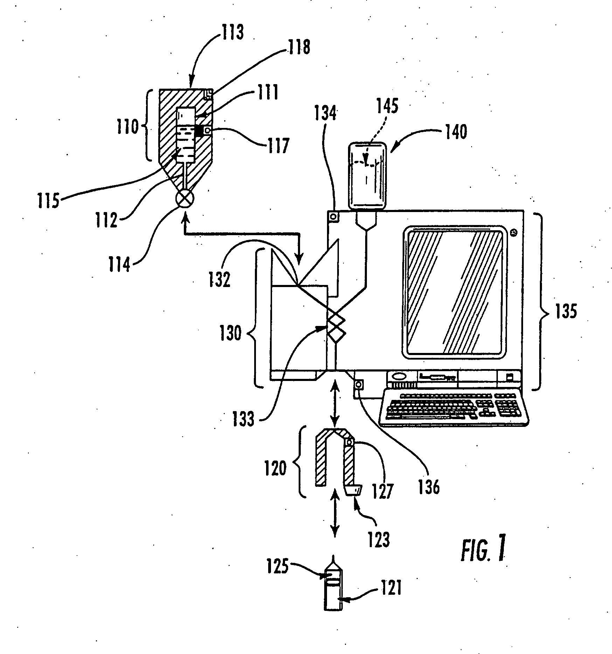

FIG. **1** shows a system for forming a mixture according to one embodiment of the present invention. The system may comprise a container **110** configured for holding a first material **115,** wherein the first material, may comprise, for instance, a radiopharmaceutical agent such as FDG, which may, in turn, produce a radiation emission. Such a first material may be produced by, for instance, a cyclotron or irradiating device, such that the container device **110** may be suitable for holding and transporting the first material **115** from a cyclotron or irradiating device to the system of the present invention.

The container device 110 may also comprise shielding 113 including, but not limited to, a lead, tungsten, polycarbonate layer, or any other layer having a thickness suitable for reducing a radiation dose received by an individual operating the present invention, wherein the radiation dose may be produced by the first material 115. One skilled in the art will appreciate that the shielding 113 may be accomplished in various manners and the shielding 113 may be altered in material, thickness, number of shielding components, and other parameters in order to provide an individual with appropriate protection from a variety of radiation types, including, but not limited to, x-rays, beta radiation, gamma radiation, and other forms of radiation, depending on the characteristics of the first material 115 contained within the container device 110. One skilled in the art will appreciate that the shielding 113 may, in some cases, be incapable of completely eliminating the absorption of radiation by an individual, however, according to the various embodiments of the present invention, the shielding 113 is suitable for reducing, and in some cases minimizing and/or eliminating the radiation dose received by an individual from a material placed within the container device 110. The shielding 113 may also be suitable for protecting an individual from other toxic and/or hazardous effects of the materials, in addition to radiation. For example, the shielding 113 may provide means for reducing an individual's exposure to toxic and/or hazardous effects that may include, but are not limited to, toxic fumes, caustic materials, and/or otherwise harmful emissions that may emanate from materials contained within the container device 110.

According to one embodiment, the container 110 may further comprise an inner container 111 for holding the first material 115. In this embodiment, the inner container 111 may be placed within the shielding 113 of the container 110 so as to provide shielding from radiation emissions that may be produced by the first material 115 which may further comprise one or more radiopharmaceutical agents or other pharmaceutical agents. The inner container 111 may further comprise a material, including but not limited to, for example, a vial composed of polymer, glass, metal, or other material suitable for containing the first material 115. Furthermore, the inner container 111 may be, in some embodiments, reusable for multiple mixing iterations in the present invention. According to other embodiments, the inner container 111 may be discarded and replaced after the first material **115** is removed from the container device **110** as described below. Furthermore, according to some embodiments, the container device **110** may be provided without an inner container **111,** such that the first material **115** is held directly within the container **110.** Further, in alternative embodiments, the container **110** may be provided with multiple inner containers **111** that may comprise additional material layers suitable for shielding an individual from the first material **115** or emissions emanating therefrom. According to embodiments of the present invention, the container device **110** (and inner container **111,** where applicable) may be suitable for minimizing handling of the first material **115** by a clinician or other operator of the present invention. Such embodiments may be especially suitable for minimizing individuals' handling of toxic, caustic, and/or otherwise harmful materials that may be components of the first material **115.**

As shown in FIG. **1****,** the container **110** may also further comprise an aperture **112** and valve **114** configured to provide a conduit such that the first material **115** contained therein may be removed or otherwise directed from the container device **110** when the container device **110** is received by the mixing device **130** as described below. The valve **114** may be located on the exterior of the shielding **113** as shown in FIG. **1****.** In other embodiments, the valve **114** may be located on the interior of the shielding **113** in communication with an opening defined in the surface of the inner container device **111.** The valve **114** may further comprise, for instance, a solenoid valve or other electromechanical mechanism, such that the valve **114** may be actuated remotely in a manner such that an operator's exposure to the first material **115,** (via handling or via a radiation dose from the first material **115)** is reduced. Alternatively, the valve **114** could be a manually actuated mechanical device such as a ball valve, stop cock or other similar device.

The container **110** and/or mixing device **130,** according to other embodiments of the present invention, may be operably engaged with a measuring device **117** (such as, for example, a first radiation measurement device, or dosimetry device **117)** suitable for determining a radiation dose emitted by the first material **115.** In such embodiments, the measuring device **117** may be located on or integrated into the container **110** such that as the container device is received by the mixing device **130** of the system, the measuring device may be in communication with a measuring port disposed on an exterior surface of the mixing device **130.** In other embodiments of the present invention, the container **110** may alternatively comprise a measuring port such that as the container device is received by the mixing device **130** of the system, the measuring port may be in communication with a measuring device **134** disposed on an exterior surface of the mixing device **130.** The measuring device **117, 134** utilized in the various embodiments of the present invention may comprise various types of radiation detection sensors, including, but not limited to, a dosimeter, Geiger counter, and/or other radiation detection means. One skilled in the art will appreciate that the measuring device **117, 134** may comprise different types of radiation detection sensors suitable for measuring the radiation dose produced by various types of, for instance, radiopharmaceutical agents, that may be utilized in the embodiments of the present invention. In addition, according to some other embodiments of the present invention, the measuring device **117, 134** may comprise a radiation detection sensor operably engaged with a digital circuit so as to provide digital information to the computer device **135** corresponding to the radioactivity level of the first material such that the predetermined ratio may be selected to produce a mixture having a predetermined radioactivity level. As described below, the computer device **135** may be operably engaged with and in communication with the dispensing device **120,** according to some embodiments of the present invention.

The container **110** may also further comprise a first memory device **118** operably engaged therewith. The first memory device **118** may be configured to receive a data set related to the first material, wherein the data within the data set related to the first material may include, but is not limited to: radiation dose of the first material at its time of fabrication, method of fabrication (such as cyclotron or irradiating device), time of fabrication, type of substance (such as, for example, FDG), and other data related to the first material. In one alternative embodiment, the data set related to the first material may be transferred to the first memory device **118** by a wire-based electronic connection such as USB port, or other physical wire connection. In other embodiments, the data set related to the first material may be transferred to the first memory device **118** by wireless methods including, but not limited to, radio frequency (RF), infra-red (IR), bluetooth or other wireless methods. The data set related to the first material stored in the first memory device **118** may be useful, for instance, in aiding the operator of the system in identifying the first material and the characteristics thereof, by, for instance, interrogating the first memory device **118,** using for instance RFID technology, wire-based electronic connection, or other suitable connection to an electronic device adapted to display the data set related to the first material upon electronically interrogating the first memory device **118**. In one alternative embodiment of the present invention, the computer device **135** may be in communication with the mixing device **130** of the present invention and may be configured to be capable of interrogating the first memory device **118** to access the data set related to the first material contained within the container device **110** so as to be capable of cross-checking, for example, the expected radiation dose of the first material (which may be calculated by the computer device **135,** for example, based on the known half-life of the first material and the radioactivity level of the first material at the time it was placed in the container device **110).** Thus, the computer device **135** may be adapted for comparing the calculated expected radiation dose of the first material with the detected radiation level of the first material that may be transmitted by the measuring device **117, 134** that may be operably engaged with the container device **110** in order to determine the substantially real-time radiation level of the first material.

According to some embodiments of the present invention, a plurality of container devices **110** may be used to contain a corresponding plurality of first materials which may comprise a variety of different materials, components, mixtures, or other compounds suitable for administering to an individual as part of a diagnostic, therapeutic, and/or medical procedure. In addition, the mixing device **130** described herein may be capable of operably engaging the plurality of container devices so as to be capable of mixing a plurality of first materials to generate a mixture containing, for example, one to ten (or more) first materials to form a mixture as described herein.

FIG. **1** also shows mixing **130** according to one embodiment of the present invention. The mixing device **130** may operably engage the container device **110** as shown in FIG. **1** such that the valve **114** and aperture **112** of the container device **110** become operably engaged with a fluid port **132** defined in an exterior surface of the mixing device **130.** The fluid port **132** may further operably engage the valve **114** such that the first material **115** may pass through the valve **114** and aperture **112** of the container device **110** and into the mixing device **130** as shown in FIG. **1****.** The fluid port **132** may further comprise, for instance, a circuit or electromechanical valve device (such as a solenoid valve, for example) configured to actuate the valve **114** of the container device **110** as it is received by the mixing device **130.**

The mixing device **130** may be configured to mix at least a portion of the first material **115** with at least a portion of the second material **145.** The first material may comprise, for instance, a radiopharmaceutical agent that is fabricated in a cyclotron or irradiating device and received from the container device **110** previously described. The second material **145** may comprise a diluent, such as, for instance, intravenous saline solution that may be suitable for diluting the first material. The second material **145** may be provided by the mixing device **130** via a second container **140** configured to hold the second material and in some embodiments, operably engaged with the mixing device. In other embodiments, the second material **145** may be provided by the mixing device **130** via a separate reservoir or other fluid system in fluid communication with the mixing device **130.** The mixing device **130** may further comprise an internal tubing set **133** configured to mix at least a portion of the first material **115** with at least a portion of the second material **145** according to a predetermined ratio, to form a mixture **125.** In one embodiment, the internal tubing set **133** may further comprise disposable polymer tubing to be replaced between each mixing cycle or at selected intervals such that, in some embodiments, a newly provided disposable sterile internal tubing set **133** may be operably engaged with the mixing device **130** prior to each subsequent mixing operation. The internal tubing set **133** may further be in communication with the fluid port **132,** container device **110,** second container device **140** and via a plurality of valve mechanisms actuated via electromechanical devices that may be controlled by the computer device **135** described generally below.

The mixing device **130,** according to some embodiments of the present invention, may further comprise a computer device **135** that may be operably engaged with the mixing device **130** and capable of cooperating with the mixing device **130** and/or measuring device **117,134** to form a mixture **125.** The mixture **125** may be formed by mixing at least a portion of the first material **115** with at least a portion of a second material **145** that may be provided by the mixing device **130,** according to a predetermined ratio to form the mixture **125.** According to some embodiments of the present invention, the computer device **135,** in cooperation with the measuring device **117,134** may determine the radiation dose emitted by, for instance, a radiopharmaceutical agent or other component of the first material **115,** and in response, adjust the predetermined ratio to provide a mixture having a selected radiation dose amount. In other embodiments, the computer device **135** may be further capable of receiving an input from an operator, wherein the input may comprise, for instance, the selected radiation dose amount. The computer device may further be capable of receiving an input comprising, for instance, an individual data set that is related to an individual to whom the mixture **125** (such as, for instance, a diluted radiopharmaceutical agent) is to be dispensed during a medical procedure such as, for example, a PET imaging procedure and/or other imaging modalities. The individual data set may comprise, for instance, weight of the individual, height of the individual, time and date of the procedure, patient identification number, and other information that may be used to calculate, for instance, an appropriate radiation dose amount. One skilled in the art will appreciate that the selected radiation dose amount of the mixture **125** may be attained by mixing a first material **115** comprising for instance, a radiopharmaceutical agent characterized by a radiation dose, with a second material, comprising for instance, an intravenous saline solution, such that the first and second materials are mixed according to a predetermined ratio. One skilled in the art will also appreciate that the selected radiation dose amount of the mixture **125** may vary depending on various factors related to the type of imaging, diagnostic, and/or therapeutic procedure being performed, the size of the individual to which the mixture **125** is being dispensed, and other factors, including, but not limited to, the radiation dose of the first material **115** prior to forming the mixture **125.**

The computer device **135** may be further adapted to communicate with a data port **136** that may be disposed, for instance, on an outer surface of the mixing device **130** so as to communicate with a second memory device **127** that may be operably engaged with a dispensing device **120.** The dispensing device **120** may hold the mixture **125** formed by the mixing device **130** of the present invention as described more specifically below. The data port **136** may further comprise, for instance, a physical electronic connection between the mixing device **130** and the second memory device **127.** According to other embodiments, the data port **136** may comprise a transceiver for sending data to the second memory device **127** via wireless methods such, as for instance, radio frequency (RF) techniques, infra-red (IR) connections, bluetooth and/or other suitable wireless methods.

FIG. 1 also shows a dispensing device **120** according to one embodiment of the present invention. The dispensing device **120** may be configured to hold the mixture **125** formed by the mixing device **130** of the system. Furthermore, in other embodiments of the present invention, the dispensing device **120** may be in communication with the mixing device **130** as shown in FIG. 1, for example, so as to reduce the handling of the mixture **125** contained within the dispensing device **120.** In other embodiments, the dispensing device **120** may be in fluid-tight fluid communication with the tubing set **133** and mixing device **130** such that the mixture **125** formed by the mixing device **130** may be directed to the dispensing device (which may further comprise shielding **123)** for dispensation thereof. Minimal handling of and exposure to the mixture **125** may be preferable in some cases, since the mixture **125,** as described above, may comprise a combination of at least a portion of the first material **115,** which may comprise, for instance a radiopharmaceutical agent, and a second material **145,** such as, for instance an intravenous saline solution. As such, the mixture **125** may be adapted to produce a selected radiation dose amount that, if unshielded, may be harmful to an operator of the system. In addition, and as described herein, the first material **115** may also contain one or more pharmaceutical agents having toxic or other adverse chemical properties such that the components of the present invention may be useful for minimizing handling of the materials and/or mixtures utilized in the various embodiments of the present invention.

As described above, the dispensing device **120** may further comprise a second memory device **127** configured to receive a data set related to the mixture **125,** wherein the data within the data set related to the mixture may include: selected radiation dose amount of the mixture at its time of formation in the mixing device **130,** classification of first material **115** used to form the mixture **125** (such as, for example, FDG), and other data related to the mixture **125.** The data set related to the mixture may be transferred to the second memory device **127** from the data port **136** by a wire-based electronic connection such as USB port, or other physical wire connection. In some embodiments, the data set related to the mixture may be transferred to the second memory device **127** from the data port **136** by wireless methods including, but not limited to, radio frequency (RF), infra-red (IR), bluetooth or other suitable wireless methods. The data set related to the mixture **125** stored in the second memory device **127** operably engaged with the dispensing device **120** may be useful, for example, in aiding the operator of the system in identifying the mixture and the characteristics thereof, by, for example, interrogating the second memory device **127,** using technologies including, but not limited to, RFID technology, wire-based electronic connection, or any other suitable connection to an electronic device adapted to display the data set related to the mixture upon electronically interrogating the second memory device **127.**

The dispensing device **120,** as shown in FIG. 1, may also comprise shielding **123** which may include, but is not limited to, a lead, tungsten, or polycarbonate layer (or any combination thereof) having a thickness suitable for shielding an operator of the system from the predetermined radiation dose amount produced by the mixture **125** contained within the dispensing device **120.** One skilled in the art will appreciate that the shielding **123** may vary in material, thickness, and other parameters in order to provide reduce an individual's exposure to a variety of radiation, including, but not limited to, x-rays, beta radiation, gamma radiation, and other radiation types having varying strengths, depending on the characteristics of the mixture **125** contained within the dispensing device **110.**

According to another embodiment, the dispensing device **120** may further comprise a cartridge device **121** for holding the mixture **125.** In this embodiment, the cartridge device **121** may be placed within the shielding **123** of the dispensing device **120** so as to provide shielding from radiation emissions that may be produced by the mixture **125** which may further comprise diluted pharmaceutical and/or radiopharmaceutical agents of various types. The cartridge device **121** may further comprise, for example, a vial or syringe composed of polymer, glass, metal, or any other material suitable for containing the mixture **125.** Furthermore, the cartridge device **121** may be, in some embodiments, reusable for multiple injection iterations in the system of the present invention. According to other embodiments, the cartridge device **121** may be disposable, and thus discarded and replaced after the mixture **125** is dispensed from the dispensing device **120** as described herein.

The dispensing device **120** may, in some embodiments, be an injection device adapted to inject at least a portion of the mixture **125** into an individual, such as, for example, an individual undergoing a PET imaging procedure. The dispensing device **120** further comprising the shielding **123** operably engaged therewith may be used, for example, with a hand-operated injection unit, adapted to inject at least a portion of the mixture **125** into an individual. The shielding **123** may protect, for example, an operator of the system, from a radiation dose emitted from the mixture **125** as at least a portion of the mixture is hand-injected into the individual. In embodiments of the present invention wherein the dispensing device **120** can also function as an injection device, one skilled in the art will appreciate that the dispensing device may further comprise, for instance, a syringe mechanism, compatible with a hand-injector, and/or power injector device **210** as described herein. In other embodiments, the dispensing device may further be suitable for dispensing the mixture to an individual for oral ingestion.

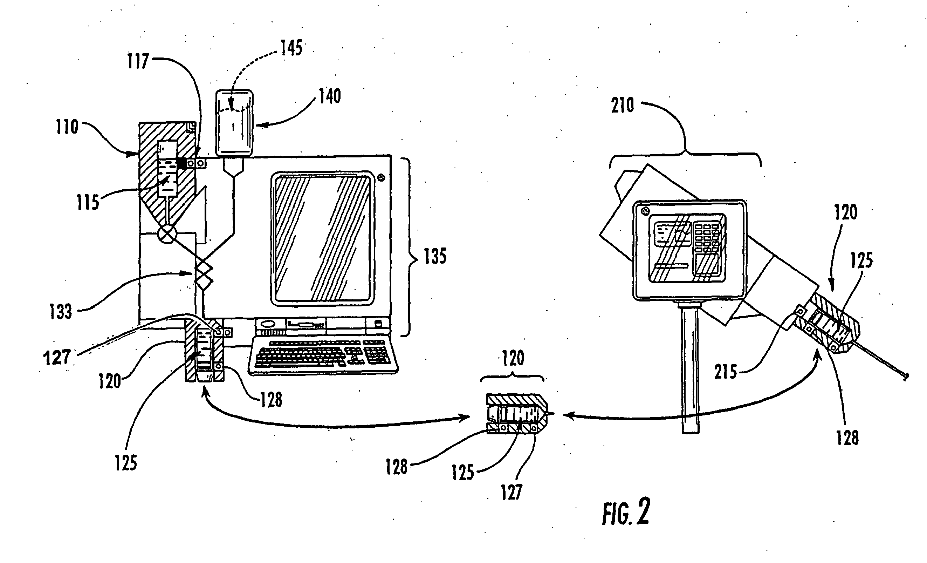

As shown in FIG. **2****,** according to other embodiments, the dispensing device **120** may be used with a power injector device **210.** In such embodiments, the power injector device **210** may receive the dispensing device **120** as shown in FIG. **2****,** wherein the power injector device **210** may actuate the dispensing device **120** to inject the mixture **125** into an individual while the operator of the system is positioned in a remote location, such as, for instance, a control room adjacent to an imaging room wherein the individual may be positioned during the course of an imaging procedure.

Furthermore, the power injector device **210** may interrogate and/or receive a data set related to the mixture **125** from, for example, the second memory device **127** that may be operably engaged with the dispensing device **120,** as described herein. In such embodiments, the power injector device **210** may identify the mixture **125** held by the dispensing device **120** so as to ensure that the selected radiation dose amount is provided to the individual, in light of the procedure type and the data related to the mixture **125.**

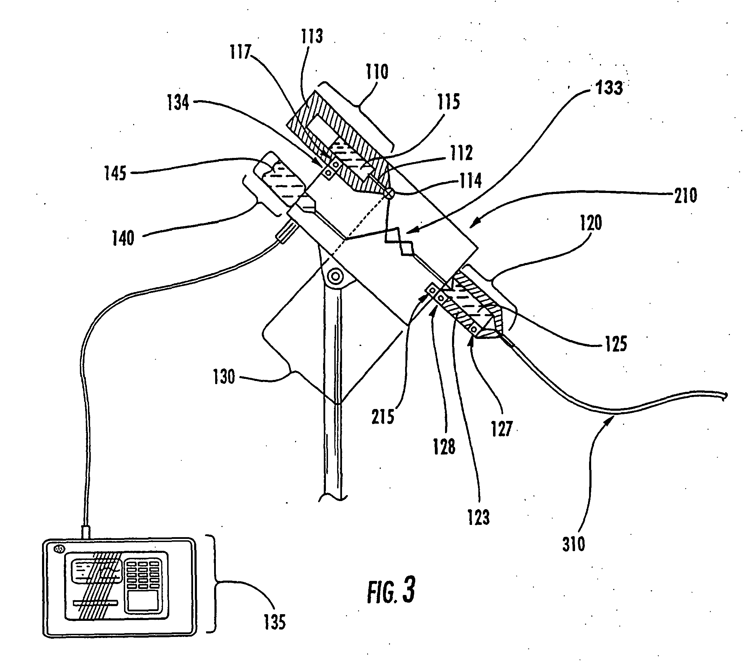

For embodiments of the present invention where the dispensing device **120** is compatible with, for instance, a power injector **210,** the dispensing device, may further comprise a measuring port **128,** compatible, for instance, with a measuring device **215** that may be operably engaged with the power injector device **210** as shown in FIG. **2****.** According to some embodiments (such as the integrated mixing device **130**/computer device **135**/automated injector device **210** embodiment shown generally in FIG. **3****),** the measuring port **128** of the dispensing device **120** may comprise a second radiation measurement device (including, but not limited to a dosimeter or digital radiation measurement sensor) for determining a radioactivity level of the mixture **125** and for transferring the determined radioactivity level of the mixture **125** to the computer device **135** such that the automated injector device **210** may be capable of dispensing a dose of the mixture to the individual corresponding to a predetermined radiation dose.

According to other embodiments of the present system, a power injector device **210** may be operably engaged with the dispensing device **120** (such as a syringe functionally encapsulated as part of the dispensing device **120).** Furthermore, the power injector device may include its own radiation measuring device **215** that may measure the radiation dose of a mixture **125** comprising, for instance, one or more pharmaceutical agents and/or radiopharmaceutical agents. The pharmaceutical agents within the mixture **125** may be held within the dispensing device **120** such that the power injector device **210** can inject at least a portion of the mixture **125** into an individual in the form of, for instance, a predetermined radiation dose amount. One skilled in the art will appreciate that the power injector device **210** may further comprise, for example, a computer device, display, control systems, and other components necessary to automatically purge a connection with the individual prior to injecting the mixture **125,** and automatically inject the mixture **125** according to a predefined flow rate and volume such that at least a portion of the mixture **125** is automatically injected into the individual.

According to some embodiments of the present invention, a plurality of dispensing devices **120** may be operably engaged with the mixing device **130** so as to be capable of receiving a corresponding plurality of mixtures that may be formed by the present invention. Thus, the mixing device **130** described herein may be capable of operably engaging the plurality of dispensing devices so as to be capable of dispensing the corresponding plurality of mixtures to one or more individuals (such a single mixing device may be capable of centrally administering mixtures to a plurality of different individuals). In other embodiments, the plurality of dispensing devices may be utilized to dispense the corresponding plurality of mixtures to a single individual as part of a therapeutic, diagnostic, and/or medical procedure requiring the dispensation of a plurality of mixtures containing, for example, one or more first materials. For example, the mixing device **130** may be operably engaged with one to ten (or more) dispensing devices **120** so as to be further capable of providing a corresponding plurality of mixtures to one or more individuals in a diagnostic, therapeutic, and/or medical procedure.

In other embodiments of the invention, such as those shown generally in FIG. **3****,** the mixing device **130** of the present invention may be integrated with the power injector device **210.** Furthermore, as described herein, the mixing device **130** may comprise a computer device **135** for controlling both the mixing device **130** and the power injector device **210** so as to inject at least a portion of the mixture **125** formed by the mixing device **130** into an individual.

As described herein, the system of the present invention may comprise a first container device **110** for holding a first material **115.** The container device **110** may thus be operably engaged with the integrated power injector device **210** and mixing device **130,** described in detail above, so as to reduce manual handling of the first material **115** and to mix at least a portion of the first material **115** with at least a portion of a second material **145** according to a predetermined ratio to form the mixture **125.** Furthermore, because the mixing device **130** and power injector device **210** are integrated in such embodiments, handling of the mixture **125** may also be reduced prior to injection of the mixture **125** into an individual. According to some embodiments of the present invention, the automated injector device **210** (and the integrated mixing device **130** included therein) may further comprise a disposable sterile tubing set **133** (or other internal tubing set **133)** for mixing at least a portion of the first material **115** (such as a radiopharmaceutical agent or other pharmaceutical agent) with at least a portion of the second material **145** (such as an intravenous saline solution) to form a mixture **125** having a predetermined ratio of first material to second material. The system of these embodiments may further comprise multiple container devices **110/140** configured to hold various types of first materials **115** and second materials **145.** The container devices **110, 140** may be engageable with the automated injector device for providing the various materials thereto with minimal handling. Furthermore, the power injector device **210** may be capable of injecting the mixture **125** into an individual via a dispensing device **120** selectively operably engaged with the power injector device **210.**

In addition, the dispensing device **120** (including, but not limited to a syringe) may be operably engaged with the power injector device **210** in a substantially fluid-tight manner so as to reduce manual handling of the mixture contained within the dispensing device **120.** For example, the dispensing device **120** may be fitted with a fluid-tight threaded connection or luer fitting for connecting (via a fluid-tight seal) to the dispensing device **210.** Furthermore, the dispensing device **120,** as described herein with respect to other system embodiments of the present invention, may also comprise a second shielding device **123** operably engaged therewith for shielding a user from emissions originating from the mixture **125** contained within the dispensing device **120.** According to some embodiments, the dispensing device **120** may be capable of operably engaging (via a luer lock or other fluid-tight connection known to those skilled in the art) an intravenous line **310** capable of carrying the mixture **125** from the dispensing device **120** to an individual (such as a human patient awaiting an imaging procedure that is dependent upon the injection of the mixture **125** in order to produce a medical image of the individual).

Furthermore, as shown in FIG. **3****,** the system of the present invention may further comprise a computer device **135** operably engaged with the automated injector device **210** and configured to cooperate with the automated injector device **210** to form the mixture **125** according to the predetermined ratio (that may be computed by the computer device **135** in response to a received dosage information input by a clinician, for example). Furthermore, the first container device **110** (containing the first material **115** (such as a radiopharmaceutical)), may further comprise a first radiation measurement device **117** operably engaged therewith for determining a radioactivity level of the first material **115** and for transferring the determined radioactivity level of the first material **115** to the computer device **135.** For example, the first radiation measurement device **117** may comprise a digital radiation sensor that is capable of measuring the radioactivity level of the first material and thereafter transferring the radioactivity level (via a data port **134** included in the automated injector device **210)** to the computer device **135** that may be operably engaged with the automated injector device **210.** Thus, the computer device **135** may be capable (using the determined radioactivity level of the first material) of producing a mixture **125** having a selected radioactivity level by selecting an appropriate predetermined ratio to produce a mixture **125** having a selected resulting radioactivity level.

According to some embodiments of the present invention, the dispensing device **120** comprises a second radiation measurement device (integrated with, for example, the measurement port **128)** operably engaged therewith for determining a radioactivity level of the mixture **125** and for transferring the determined radioactivity level of the mixture to the computer device **135** such that the power injector device **210** may be capable of dispensing a dose of the mixture **125** to the individual corresponding to a predetermined radiation dose. In one alternative embodiment, the computer device **135** may, in response to the determined radioactivity level of the mixture **125,** direct the automated injector device **210** to adjust the overall amount of the mixture **125** that is administered to the individual via the dispensing device **120.** Furthermore, the computer device **135** may also direct the automated injector device **210** to adjust the predetermined ratio of the mixture **125** by increasing the amount of first material **115** and/or second material **145** that is present in the resultant mixture **125** until a selected radiation dose is achieved and confirmed by the second radiation measurement device **128.**

Additionally, such a power injector device **210** may further comprise, for instance, multiple dispensing cartridges or syringes containing, mixtures, flushing agents (including, but not limited to, intravenous saline solution, water, or other suitable diluents), first materials **115** and/or second materials **145.** The outlet of each cartridge may be in fluid communication to the line **310** delivering the pharmaceutical agent, radiopharmaceutical agent, or other mixture **125** to the patient for diagnostic, therapeutic, imaging, dilution, and/or flushing purposes. Such a line would assure that any volume of material in the tube going to the patient is effectively utilized.

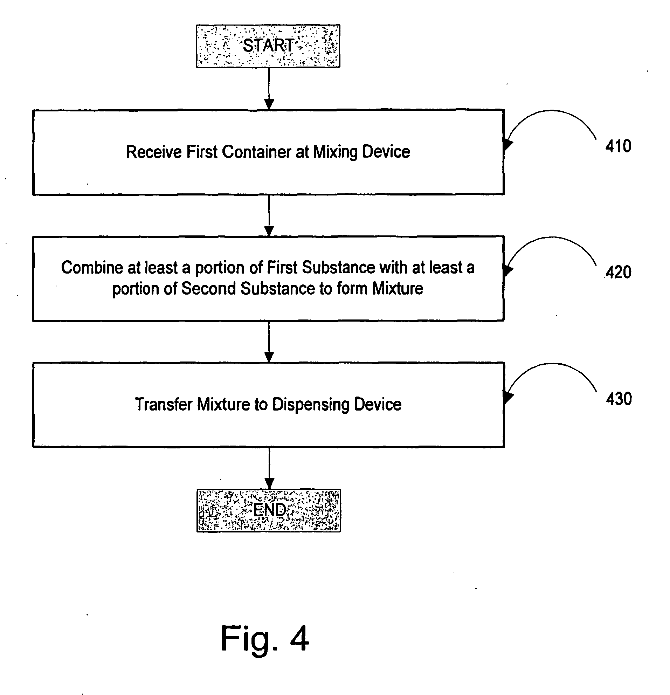

FIGS. **4-8** illustrate several non-limiting exemplary method embodiments for forming a mixture **125** by mixing, for instance, a first material **115** with a second material **145** using a mixing device **130** such as that disclosed generally above with regard to the system embodiments of the present invention. The resulting mixture **125** may also comprise other ingredients as well. First, as shown in FIG. **4****,** in step **410,** the container device **110,** holding the first material **115,** is received by the mixing device **130.** The receiving step **410** may further comprise, for instance, measuring the radiation dose of a first material **115** held in the container device **110** using, for instance, a measuring device **117, 134** (including, but not limited to a dosimeter or digital radiation sensor capable of communicating with a computer device **135** that may be operably engaged and/or in communication with the mixing device **130).**

In step **420,** the mixing device **130** mixes at least a portion of the first material **115** with at least a portion of the second material **145** according to a predetermined ratio to form the mixture **125.** The mixing step **420** may further comprise, for instance, the steps of receiving the dispensing device **120** configured to hold the mixture **125;** determining the predetermined ratio using a selected radiation dose amount input by an operator of the system; and determining the radiation dose of the first material **115** measured by, for instance, the measuring device **117, 134** in step **410.** The mixing step **420** includes, for instance, mixing at least a portion of the first material **115** with at least a portion of the second material **145** according to a predetermined ratio in the internal tubing set **133** of the mixing device **130.**

Step **430** comprises transferring the mixture **125** to the dispensing device **120.** Step **430** may occur automatically as the mixing device **130** forms the mixture **125** and transfers the mixture to the dispensing device **120** received in the mixing device **130** as shown in FIG. **1****.** For example, step **430** may comprise filling a shielded syringe or other dispensing device **120** with a mixture **125** formed by the mixing device **130** or automated injector device **210** of the present invention.

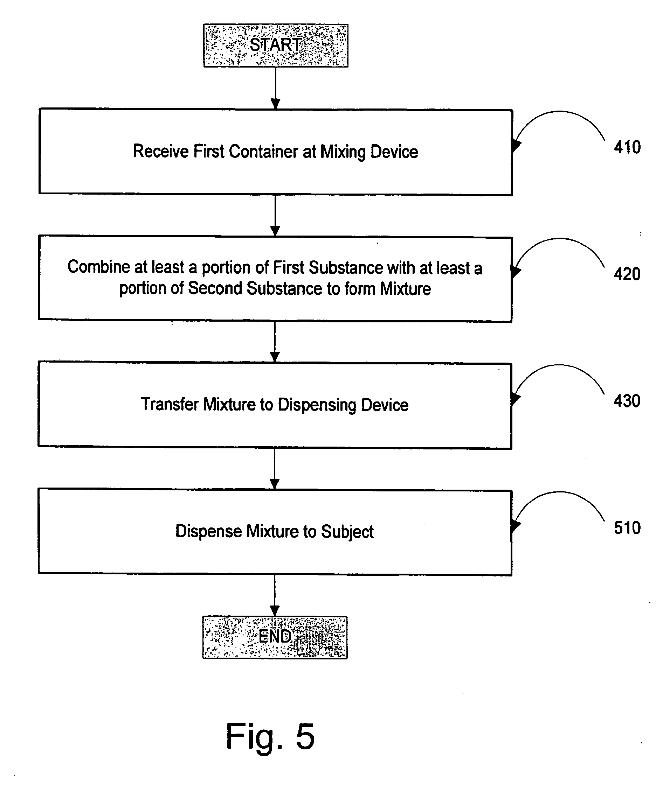

As shown in FIG. **5**, a method may comprise dispensing the mixture **125** to an individual (including, but not limited to a patient awaiting a subsequent medical imaging procedure) as shown generally in step **510.** Step **510** may comprise actuating an automated injector device **210** (including, but not limited to the automated injector device **210** shown in FIG. **3****)** to inject the mixture **125** (contained in a syringe or other dispensing device **120)** into an individual. Step **510** may comprise injecting the mixture **125** directly from the dispensing device **125** via a needle and/or indirectly via an intravenous line **310** as shown in FIG. **3** or other suitable means). A separate mixing device **130** and automated injector device **210** (including, but not limited to those shown generally in FIG. **2****)** may be utilized. The method may further comprise disengaging the dispensing device **120** from the mixing device **130** (subsequent to the production of the mixture) and subsequently operably engaging the dispensing device **120** with the separate automated injector device **210** for automatically injecting the mixture into an individual (step **510,** for example). The dispensing step **510** may further comprise engaging the dispensing device **120** described above with a hand-injector and/or syringe device for manual injection into an individual.

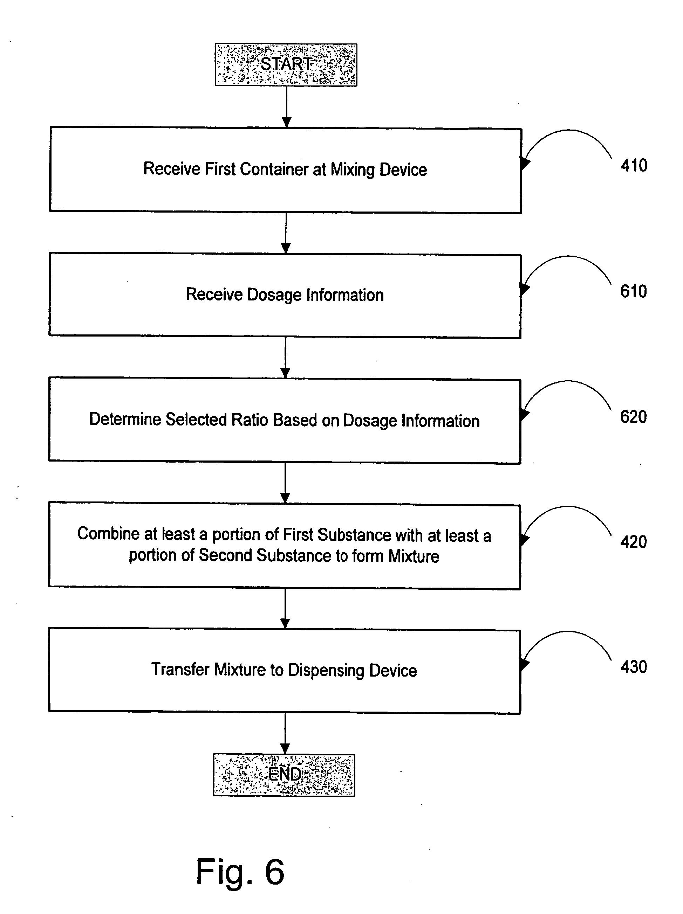

Referring to FIG. **6****,** the method may further comprise several dosage calculation steps prior to controlling the mixing device **130** (or automated injector device **210,** as shown in FIG. **3****)** to mix the first material **115** and second material **145** to form the mixture **125.** For example, step **610** comprises receiving dosage information corresponding to a selected dose of the mixture **125** to be dispensed to an individual. For example, step **610** may comprise receiving (via the computer device **135** or other user interface in communication with the mixing device **130** and/or automated injector device **210)** dosage information (including, but not limited to a total desired radiation dose in millicuries) that may be input by a clinician or health physicist prior to the initiation of the production of the mixture **125** to be administered to the individual. Furthermore, as shown in step **620,** the method may further comprise determining the predetermined ratio prior to the controlling step based on the received dosage information. The computer device **135,** mixing device **130,** and/or automated injector device 210 may be configured to be capable of determining the predetermined ratio of first material **115** to second material **145** (including, but not limited to, saline, water, or other suitable diluent) to form a mixture **125** that is capable of providing the selected dosage based on the radioactivity, half-life, and decay time information for a given radiopharmaceutical that may be present in the first material **115.**

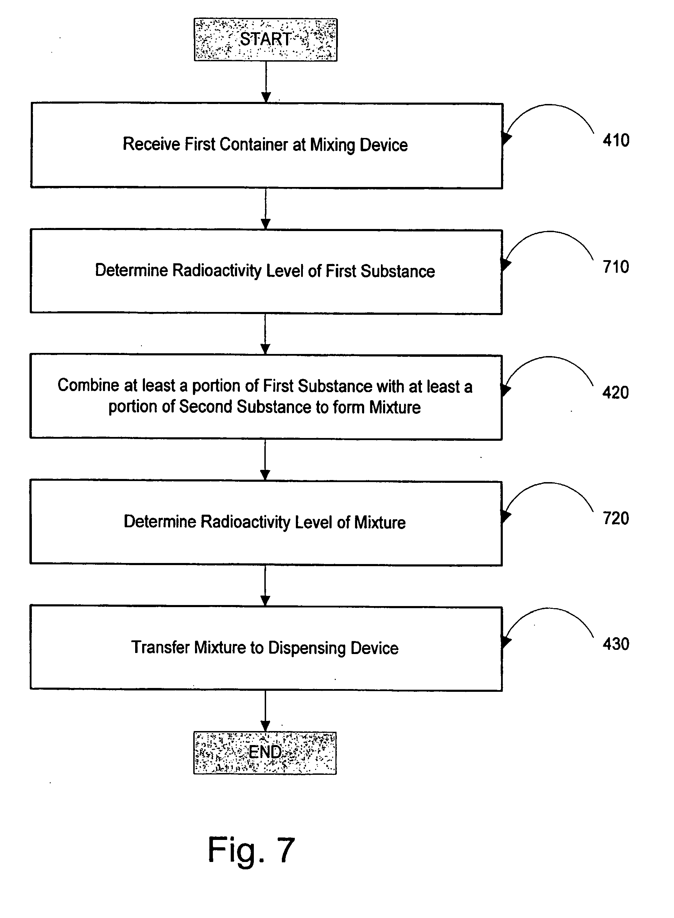

Thus, as shown in FIG. **7****,** the computer device **135** of the system embodiments of the present invention may interrogate the first radiation measurement device **117** and/or the first memory device **118** included as part of the first container device in order to determine baseline radiation, half-life, and/or identification information related to the first material **115,** as shown in step **710.** Thus according to method step **710,** the computer device **135** may be capable of determining the predetermined ratio based on a selected dosage or dose rate that may be input by an individual and received as part of step **610** as well as the baseline radiation, half-life, and/or identification information related to the first material **115.** Furthermore, as shown in FIG. **7****,** the method of the present invention may also comprise determining a radioactivity level of the mixture **125** prior to the directing step (step **430,** for example) such that the mixture **125** may be dispensed to an individual so as to expose the individual to a selected radiation dose that may be input by an individual, including but not limited to a clinician or heath physicist, as part of step **610** (shown in FIG. **6****).**

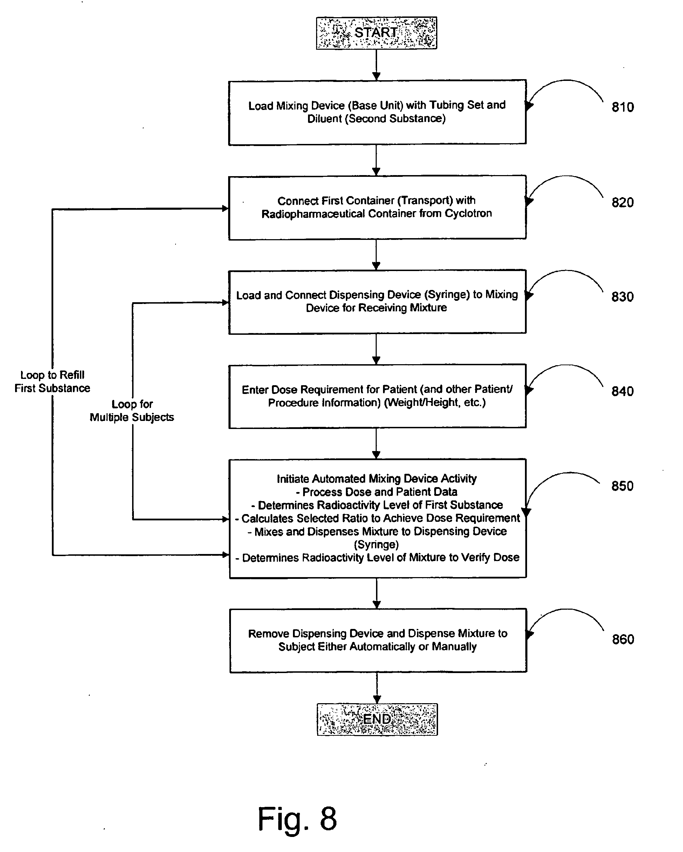

FIG. **8** shows an exemplary embodiment of the present invention suitable for mixing a first material comprising a radiopharmaceutical with a second material comprising a diluent. Step **510** comprises loading the mixing device **130** (denoted as the "base unit") with diluent (the second material **145)** and an internal tubing set **133** (such as a disposable sterile tubing set **133,** as described herein). Step **820** comprises connecting the container device **110** with a cyclotron container, containing, for instance, radiopharmaceutical agents. Step **830** comprises loading and connecting the dispensing device **120** (including, but not limited to a fluid-tight syringe) to the mixing device **130.** In step **840,** data related to the imaging procedure may be entered into the computer device **135** of the present invention. Step **850** which may be performed by the mixing device **130** in cooperation with the computer device **135** operably engaged therewith, may comprise automatically processing the data related to the imaging procedure, measuring a radiation dose emitted by the radiopharmaceutical (contained, for example in the first material **115** and measured by the first radiation measurement device **117** operably engaged with the first container device **110),** determining the predetermined ratio to achieve a selected radiation dose amount, automatically mixing the first **115** and second materials **145** to form the mixture **125,** and verifying the selected radiation dose amount emitted by the mixture **125.** Step **860** comprises removing the dispensing device **120** from the mixing device **130** and dispensing the mixture **125** to an individual as part of a medical procedure, including but not limited to: a PET imaging procedure, a CT imaging procedure, an alternate medical imaging procedure, a therapeutic procedure, a diagnostic procedure, and/or other suitable medical procedures requiring the injection of the mixture **125.**

In addition to providing systems and methods, the present invention also provides computer program products for performing the operations described above. The computer program products have a computer readable storage medium having computer readable program code means embodied in the medium. With reference to Figure **1****,** the computer readable storage medium may be included as part of the computer device **135** in communication with the mixing device **130** and/or automated injector device **210** (as shown generally in FIG. **3****),** and may implement the computer readable program code means to perform the above discussed operations.

Figures **4-8** are non-limiting block diagrams, flowcharts and control flow illustrations of methods, systems and program products according to embodiments of the invention. It will be understood that each block or step of the block diagrams, flowcharts and control flow illustrations, and combinations of blocks in the block diagrams, flowcharts and control flow illustrations, can be implemented by computer program instructions. These computer program instructions may be loaded onto a computer (including, but not limited to the computer device **135** in communication with the mixing device **130** and/or automated injector device **210** described herein with respect to the embodiments of the present invention) or other programmable apparatus to produce a machine, such that the instructions which execute on the computer or other programmable apparatus form means for implementing the functions specified in the block diagrams, flowcharts or control flow block(s) or step(s). These computer program instructions may also be stored in a computer-readable memory that can direct a computer or other programmable apparatus to function in a particular manner, such that the instructions stored in the computer-readable memory may produce an article of manufacture including instruction means which can implement the function specified in the block diagrams, flowcharts or control flow block(s) or step(s). The computer program instructions may also be loaded onto a computer or other programmable apparatus, among other things, to cause a series of operational steps to be performed on the computer or other programmable apparatus. This may produce a computer implemented process such that the instructions which execute on the computer or other programmable apparatus provide steps for implementing the functions specified in the block diagrams, flowcharts or control flow block(s) or step(s).

Accordingly, blocks or steps of the block diagrams, flowcharts or control flow illustrations support, among other things, combinations of means for performing the specified functions, combinations of steps for performing the specified functions and program instruction means for performing the specified functions. It will also be understood that each block or step of the block diagrams, flowcharts or control flow illustrations, and combinations thereof, can be implemented by special purpose hardware-based computer systems which perform the specified functions or steps, or combinations of special purpose hardware and computer instructions.

Other modifications and other embodiments of the invention set forth herein will come to mind to one skilled in the art to which this invention pertains having the benefit of the teachings presented in the foregoing descriptions and on the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

Further, throughout the description, where compositions are described as having, including, or comprising specific components, or where processes systems or methods are described as having, including, or comprising specific steps, it is contemplated that compositions or the present invention may also consist essentially or, or consist of the recited components, and that the processes or methods of the present invention also consist essentially or consist of the recited steps. Further, it should be understood that the order of steps or order of performing certain actions are immaterial so long as the invention remains operable. Moreover, two or more steps or actions may be conducted simultaneously with respect to the invention disclosed herein.

## Claims

1. A system for dispensing a mixture into an individual, the system comprising:
at least one container, suitable for holding one or more materials;
at least one dispensing device;
at least one mixing device for engaging the one or more containers so as to reduce handling of a first one or more materials contained therein and to mix at least a portion of the first one or more materials with at least a portion of a second one or more materials according to a predetermined ratio to form a mixture, the at least one mixing device being capable of directing the mixture to the at least one dispensing device for dispensation thereof into the individual;
a computer device operably engaged with the at least one mixing device and capable of determining the predetermined ratio and cooperating with the at least one mixing device to form the mixture according to the predetermined ratio; and
a first radiation measurement device operably engaged with the at least one container for determining a radioactivity level of the first one or more materials or second one or more materials and for transferring the determined radioactivity level of the first one or more materials or second one or more materials to the computer device such that the predetermined ratio may be selected to produce a mixture having a selected radioactivity level.

2. A system according to Claim 1, wherein the at least one mixing device comprises a disposable sterile tubing set for mixing at least a portion of the first one or more materials with at least a portion of the second one or more materials.

3. A system according to Claim 1, wherein the at least one container comprises a first memory device configured to be capable of receiving and storing a data set containing data related to the first one or more materials and wherein the at least one mixing device is further capable of interrogating the first memory device to access the data set.

4. A system according to Claim 1, further comprising a second container device for holding the second one or more materials and to be engageable with the at least one mixing device for providing the second one or more materials thereto.

5. A system according to Claim 1, wherein the at least one mixing device is further capable of engaging the at least one dispensing device so as to supply the mixture thereto.

6. A system according to Claim 1, wherein the at least one dispensing device comprises an injection device adapted to inject at least a portion of the mixture into the individual.

7. A system according to Claim 6, wherein the at least one dispensing device is capable of operably engaging an automated injector device for injecting at least a portion of the mixture into the individual.

8. A system according to Claim 1, wherein the computer device is further capable of receiving dosage information, the computer device being further capable of determining the predetermined ratio from the dosage information received thereby.

9. A system according to Claim 1, wherein the at least one dispensing device comprises a second radiation measurement device operably engaged therewith for determining a radioactivity level of the mixture and for transferring the determined radioactivity level of the mixture to the computer device such that the at least one dispensing device may be capable of dispensing a dose of the mixture to the individual corresponding to a selected radiation dose.

10. A system according to Claim 1, wherein the at least one container further comprises a first shielding device operably engaged therewith for shielding a user from the first one or materials or the second one or more materials contained therein.

11. A system according to Claim 1, wherein the at least one dispensing device further comprises a second shielding device operably engaged therewith for shielding a user from the mixture contained therein.

12. A system according to Claim 1, wherein the dispensing device comprises a syringe.

13. A system for dispensing a mixture into an individual, the system comprising:
a first container for holding a first material;
an automated injector device capable of engaging the container so as to reduce manual handling of the first material contained therein and to mix at least a portion of the first material with at least a portion of a second material according to a predetermined ratio to form a mixture, the automated injector device further capable of injecting the mixture into the individual by a dispensing device selectively operably engaged with the automated injector device;
a computer device operably engaged with the automated injector device, the computer device capable of determining the predetermined ratio and configured to cooperate with the automated injector device to form the mixture according to the predetermined ratio; and
a first radiation measurement device operably engaged with the first container device for determining a radioactivity level of the first material or the second material and for transferring the determined radioactivity level of the first material or second material to the computer device such that the predetermined ratio may be selected to produce a mixture having a selected radioactivity level.

14. A system according to Claim 13, wherein the automated injector device comprises a disposable sterile tubing set for mixing at least a portion of the first material with at least a portion of the second material.

15. A system according to Claim 13, wherein the first container device comprises a first memory device capable of receiving and storing a data set containing data related to the first material and wherein the automated injector device is further capable of interrogating the first memory device to access the data set.

16. A system according to Claim 13, wherein the dispensing device is capable of operably engaging the automated injector device in a substantially fluid-tight manner to reduce manual handling of the mixture contained therein.

17. A system according to Claim 13, wherein the dispensing device comprises a syringe.

18. A system according to Claim 13, further comprising a third container device for holding the second material and for selectively engaging the automated injector device for providing the second material thereto.

19. A system according to Claim 13, wherein the computer device is further capable of receiving dosage information, the computer device being further capable of determining the predetermined ratio from the dosage information received thereby.

20. A system according to Claim 13, wherein the dispensing device comprises a second radiation measurement device operably engaged therewith for determining a radioactivity level of the mixture and for transferring the determined radioactivity level of the mixture to the computer device such that the automated injector device may be capable of dispensing a dose of the mixture to the individual corresponding to a selected radiation dose.

21. A system according to Claim 13, wherein the first container device further comprises a first shielding device operably engaged therewith for shielding a user from the first material or the second material contained therein.

22. A system according to Claim 13, wherein the dispensing device further comprises a second shielding device operably engaged therewith for shielding a user from the mixture contained therein.

23. A method of preparing one or more mixtures for dispensation into an individual, the method comprising:
operably engaging at least one container for holding a first one or more materials with at least one mixing device, so as to provide the first one or more materials to the mixing device and reduce handling of the first one or more materials contained in the container device;
determining a radioactivity level of the first one or more materials or a second one or more materials;
mixing, with the mixing device, at least a portion of the first one or more materials and at least a portion of the second one or more materials according to a predetermined ratio to form the one or more mixtures having a predetermined radioactivity level based at least in part on the determined radioactivity level; and
directing the one or more mixtures to a dispensing device, the dispensing device being capable of dispensing the mixture into the individual.

24. A method according to Claim 23, further comprising operably engaging the dispensing device with an automated injector device for automatically injecting the mixture into the individual.

25. A method according to Claim 23, further comprising receiving dosage information corresponding to a selected dose of the mixture to be dispensed to the individual.

26. A method according to Claim 25, further comprising determining the predetermined ratio prior to the mixing step based on the received dosage information.

27. A method according to Claim 23, further comprising determining a radioactivity level of the mixture prior to the directing step such that the mixture may be dispensed to the individual so as to expose the individual to a selected radiation dose.

28. A computer program product capable of controlling a mixing device to form a mixture, the computer program product comprising a computer-readable storage medium having computer-readable program code portions stored therein, the computer-readable program code portions comprising:
a first executable portion for operably engaging a container device for holding a first material with the mixing device so as to supply the first material to the mixing device;
a second executable portion for determining a radioactivity level of the first material or a second material;
a third executable portion for controlling the mixing device to mix at least a portion of the first material and at least a portion of the second material according to a predetermined ratio to form the mixture having a predetermined radioactivity level based at least in part on the determined radioactivity level; and
a fourth executable portion for directing the mixture to a dispensing device, the dispensing device being capable of dispensing the mixture into an individual.

29. A computer program product according to Claim 28, further comprising a fifth executable portion for dispensing the mixture by injecting the mixture into an individual with the dispensing device via an automated injector device.

30. A computer program product according to Claim 28, further comprising a sixth executable portion for receiving dosage information corresponding to a selected dose of the mixture to be dispensed into the individual.

31. A computer program product according to Claim 30, further comprising a seventh executable portion for determining the predetermined ratio prior to the second executable portion based on the received dosage information.

32. A computer program product according to Claim 28, further comprising an eighth executable portion for determining a radioactivity level of the mixture prior to the third executable portion such that the mixture may be dispensed to the individual so as to expose the individual to a selected radiation dose.

## Patentansprüche

1. System zum Ausgeben eines Gemisches in ein Individuum, wobei das System umfasst:
mindestens einen Behälter, welcher geeignet ist, um eine oder mehrere Materialien aufzunehmen;
mindestens eine Ausgabevorrichtung;
mindestens eine Mischvorrichtung, um den einen oder die mehreren Behälter zu koppeln, um ein Handhaben von einer oder von mehreren Materialien zu verringern, welche darin enthalten sind, und um mindestens einen Teil des ersten oder der mehreren ersten Materialien mit mindestens einem Teil eines zweiten oder mehreren zweiten Materialien entsprechend einem vorbestimmten Verhältnis zu mischen, um ein Gemisch auszubilden, wobei die mindestens eine Mischvorrichtung in der Lage ist, das Gemisch zu der mindestens einen Ausgabevorrichtung zu führen, um es in das Individuum auszugeben;
eine Computervorrichtung, welche betriebsbereit mit der mindestens einen Mischvorrichtung gekoppelt ist und in der Lage ist, das vorbestimmte Verhältnis zu bestimmen und mit der mindestens einen Mischvorrichtung derart zusammenwirkt, dass das Gemisch gemäß dem vorbestimmten Verhältnis ausgebildet wird; und
eine erste Strahlungsmessvorrichtung, welche betriebsbereit mit dem mindestens einen Behälter gekoppelt ist, um ein Radioaktivitätsniveau des ersten oder der mehreren ersten Materialien oder des zweiten oder der mehreren zweiten Materialien zu bestimmen und um das bestimmte Radioaktivitätsniveau des ersten oder der mehreren ersten Materialien oder des zweiten oder der mehreren zweiten Materialien an die Computervorrichtung zu übertragen, so dass das vorbestimmte Verhältnis derart ausgewählt werden kann, dass ein Gemisch mit einem ausgewählten Radioaktivitätsniveau erzeugt wird.

2. System nach Anspruch 1, wobei die mindestens eine Mischvorrichtung einen wegwerfbaren sterilen Rohrleitungssatz umfasst, um zumindest einen Teil des ersten oder der mehreren zweiten Materialien mit zumindest einem Teil des zweiten oder der mehreren zweiten Materialien zu mischen.

3. System nach Anspruch 1, wobei der mindestens eine Behälter eine erste Speichervorrichtung umfasst, welche derart ausgestaltet ist, dass sie in der Lage ist, einen Datensatz aufzunehmen und zu speichern, welcher Daten bezüglich des ersten oder der mehreren ersten Materialien enthält, und wobei die mindestens eine Mischvorrichtung darüber hinaus in der Lage ist, die erste Speichervorrichtung derart abzufragen, dass sie Zugang zu dem Datensatz erhält.

4. System nach Anspruch 1, darüber hinaus eine zweite Behältervorrichtung umfassend, um das zweite oder die mehreren zweiten Materialien aufzunehmen und um mit der mindestens einen Mischvorrichtung koppelbar zu sein, um das zweite oder die mehreren zweiten Materialien dazu bereitzustellen.

5. System nach Anspruch 1, wobei die mindestens eine Mischvorrichtung darüber hinaus in der Lage ist, mit der mindestens einen Ausgabevorrichtung gekoppelt zu werden, um das Gemisch dazu zuzuliefern.

6. System nach Anspruch 1, wobei die mindestens eine Ausgabevorrichtung eine Injektionsvorrichtung umfasst, welche derart ausgestaltet ist, dass sie zumindest einen Teil des Gemisches in das Individuum injiziert.

7. System nach Anspruch 6, wobei die mindestens eine Ausgabevorrichtung in der Lage ist, betriebsbereit mit einer automatisierten Injektionsvorrichtung gekoppelt zu sein, um zumindest einen Teil des Gemisches in das Individuum zu injizieren.

8. System nach Anspruch 1, wobei die Computervorrichtung darüber hinaus in der Lage ist, eine Dosierungsinformation aufzunehmen, wobei die Computervorrichtung darüber hinaus in der Lage ist, das vorbestimmte Verhältnis von der Dosierungsinformation, welche sie dabei aufgenommen hat, zu bestimmen.

9. System nach Anspruch 1, wobei die mindestens eine Ausgabevorrichtung eine zweite Strahlungsmessvorrichtung umfasst, welche betriebsbereit damit gekoppelt ist, um ein Radioaktivitätsniveau des Gemisches zu bestimmen und das bestimmte Radioaktivitätsniveau des Gemisches zu der Computervorrichtung zu übertragen, so dass die mindestens eine Ausgabevorrichtung in der Lage ist, entsprechend einer ausgewählten Strahlungsdosis eine Dosis des Gemisches an das Individuum auszugeben.

10. System nach Anspruch 1, wobei der mindestens eine Behälter darüber hinaus eine erste Abschirmungsvorrichtung umfasst, welche damit betriebsbereit gekoppelt ist, um eine Bedienperson vor dem ersten oder den mehreren ersten Materialien oder dem zweiten oder den mehreren zweiten Materialien, welche darin enthalten sind, abzuschirmen.

11. System nach Anspruch 1, wobei die mindestens eine Ausgabevorrichtung darüber hinaus eine zweite Abschirmungsvorrichtung umfasst, welche damit betriebsbereit gekoppelt ist, um eine Bedienperson vor dem Gemisch, welches darin enthalten ist, abzuschirmen.

12. System nach Anspruch 1, wobei die Ausgabevorrichtung eine Spritze umfasst.

13. System zum Ausgeben eines Gemisches in ein Individuum, wobei das System umfasst:
einen ersten Behälter, um ein erstes Material aufzunehmen;
eine automatisierte Injektionsvorrichtung, welche in der Lage ist, mit dem Behälter gekoppelt zu sein, um eine manuelle Handhabung des ersten Materials, welches darin enthalten ist, zu verringern, und um zumindest einen Teil des ersten Materials mit zumindest einem Teil eines zweiten Materials entsprechend einem vorbestimmten Verhältnis zu mischen, um ein Gemisch auszubilden, wobei die automatisierte Injektionsvorrichtung darüber hinaus in der Lage ist, das Gemisch in das Individuum zu injizieren, indem eine Ausgabevorrichtung selektiv betriebsbereit mit der automatisierten Injektionsvorrichtung gekoppelt ist;
eine Computervorrichtung, welche betriebsbereit mit der automatisierten Injektionsvorrichtung gekoppelt ist, wobei die Computervorrichtung in der Lage ist, das vorbestimmte Verhältnis zu bestimmen, und derart ausgestaltet ist, dass sie mit der automatisierten Injektionsvorrichtung derart zusammenwirkt, dass das Gemisch entsprechend dem vorbestimmten Verhältnis ausgebildet wird; und
eine erste Strahlungsmessvorrichtung, welche betriebsbereit mit der ersten Behältervorrichtung gekoppelt ist, um ein Radioaktivitätsniveau des ersten Materials oder des zweiten Materials zu bestimmen und um das bestimmte Radioaktivitätsniveau des ersten Materials oder des zweiten Materials zu der Computervorrichtung zu übertragen, so dass das vorbestimmte Verhältnis derart ausgewählt werden kann, dass ein Gemisch mit einem ausgewählten Radioaktivitätsniveau erzeugt wird.

14. System nach Anspruch 13, wobei die automatisierte Injektionsvorrichtung einen wegwerfbaren sterilen Rohrleitungssatz umfasst, um zumindest einen Teil des ersten Materials mit zumindest einem Teil des zweiten Materials zu mischen.

15. System nach Anspruch 13, wobei die erste Behältervorrichtung eine erste Speichervorrichtung umfasst, welche in der Lage ist, einen Datensatz aufzunehmen und zu speichern, welcher Daten bezüglich des ersten Materials enthält, und wobei die automatisierte Injektionsvorrichtung darüber hinaus in der Lage ist, die erste Speichervorrichtung derart abzufragen, dass sie Zugang zu dem Datensatz erhält.

16. System nach Anspruch 13, wobei die Ausgabevorrichtung in der Lage ist, mit der automatisierten Injektionsvorrichtung in einer im Wesentlichen flüssigkeitsfesten Weise betriebsbereit gekoppelt zu sein, um eine manuelle Handhabung des Gemisches, welches darin enthalten ist, zu verringern.

17. System nach Anspruch 13, wobei die Ausgabevorrichtung eine Spritze umfasst.

18. System nach Anspruch 13, darüber hinaus eine dritte Behältervorrichtung umfassend, um das zweite Material aufzunehmen und um selektiv mit der automatisierten Injektionsvorrichtung gekoppelt zu sein, um das zweite Material dazu bereitzustellen.

19. System nach Anspruch 13, wobei die Computervorrichtung darüber hinaus in der Lage ist, eine Dosierungsinformation aufzunehmen, wobei die Computervorrichtung darüber hinaus in der Lage ist, das vorbestimmte Verhältnis von der Dosierungsinformation, welche **dadurch** aufgenommen worden ist, zu bestimmen.

20. System nach Anspruch 13, wobei die Ausgabevorrichtung eine zweite Strahlungsmessvorrichtung umfasst, welche damit betriebsbereit gekoppelt ist, um ein Radioaktivitätsniveau des Gemisches zu bestimmen und das bestimmte Radioaktivitätsniveau des Gemisches zu der Computervorrichtung zu übertragen, so dass die automatisierte Injektionsvorrichtung in der Lage ist, eine Dosis des Gemisches entsprechend einer ausgewählten Strahlungsdosis an das Individuum auszugeben.

21. System nach Anspruch 13, wobei die erste Behältervorrichtung darüber hinaus eine erste Abschirmungsvorrichtung umfasst, welche damit betriebsbereit gekoppelt ist, um eine Bedienperson vor dem ersten Material oder vor dem zweiten Material, welche darin enthalten sind, abzuschirmen.

22. System nach Anspruch 13, wobei die Ausgabevorrichtung darüber hinaus eine zweite Abschirmungsvorrichtung umfasst, welche damit betriebsbereit gekoppelt ist, um eine Bedienperson vor dem Gemisch, welches darin enthalten ist, abzuschirmen.

23. Verfahren zur Herstellung eines oder mehrerer Gemische zur Ausgabe in ein Individuum, wobei das Verfahren umfasst:
betriebsbereites Koppeln von mindestens einem Behälter, um ein erstes oder mehrere erste Materialien mit mindestens einer Mischvorrichtung aufzunehmen, um so das erste oder die mehreren ersten Materialien der Mischvorrichtung bereitzustellen und eine Handhabung des ersten oder der mehreren ersten Materialien, welche in der Behältervorrichtung enthalten sind, zu verringern;
Bestimmen eines Radioaktivitätsniveaus des ersten oder der mehreren ersten Materialien oder des zweiten oder der mehrerer zweiten Materialien;
Mischen zumindest eines Teils des ersten oder der mehreren ersten Materialien und zumindest eines Teils des zweiten oder der mehreren zweiten Materialien entsprechend einem vorbestimmten Verhältnis mit der Mischvorrichtung, um das eine oder die mehreren Gemische auszubilden, welche ein vorbestimmtes Radioaktivitätsniveau basierend zumindest teilweise auf dem bestimmten Radioaktivitätsniveau aufweisen; und
Führen des einen oder der mehreren Gemische zu einer Ausgabevorrichtung, wobei die Ausgabevorrichtung in der Lage ist, das Gemisch in das Individuum auszugeben.

24. Verfahren nach Anspruch 23, darüber hinaus ein betriebsbereites Koppeln der Ausgabevorrichtung mit einer automatisierten Injektionsvorrichtung umfassend, um das Gemisch automatisch in das Individuum zu injizieren.

25. Verfahren nach Anspruch 23, darüber hinaus ein Aufnehmen einer Dosierungsinformation umfassend, welche einer ausgewählten Dosis des Gemisches entspricht, welche an das Individuum auszugeben ist.

26. Verfahren nach Anspruch 25, darüber hinaus ein Bestimmen des vorbestimmten Verhältnisses vor dem Mischschritt umfassend, welches auf der aufgenommenen Dosierungsinformation basiert.

27. Verfahren nach Anspruch 23, darüber hinaus ein Bestimmen eines Radioaktivitätsniveaus des Gemisches vor dem Zuführungsschritt umfassend, so dass das Gemisch derart an das Individuum ausgegeben werden kann, dass das Individuum einer ausgewählten Strahlungsdosis ausgesetzt ist.

28. Computerprogrammprodukt, welches in der Lage ist, eine Mischvorrichtung zu steuern, um ein Gemisch auszubilden, wobei das Computerprogrammprodukt ein von einem Computer lesbares Speichermedium umfasst, welches von einem Computer lesbare Programmcodeabschnitte aufweist, welche darin gespeichert sind, wobei die von einem Computer lesbaren Programmcodeabschnitte umfassen:
einen ersten ausführbaren Abschnitt, um eine Behältervorrichtung betriebsbereit zu koppeln, um ein erstes Material mit der Mischvorrichtung aufzunehmen, um das erste Material der Mischvorrichtung zuzuführen;
einen zweiten ausführbaren Abschnitt, um ein Radioaktivitätsniveau des ersten Materials oder eines zweiten Materials zu bestimmen;
einen dritten ausführbaren Abschnitt, um die Mischvorrichtung derart zu steuern, dass sie zumindest einen Teil des ersten Materials und zumindest einen Teil des zweiten Materials entsprechend einem vorbestimmten Verhältnis mischt, um das Gemisch mit einem vorbestimmten Radioaktivitätsniveau, welches zumindest teilweise auf dem bestimmten Radioaktivitätsniveau basiert, auszubilden; und
einen vierten ausführbaren Abschnitt, um das Gemisch zu einer Ausgabevorrichtung zu führen, wobei die Ausgabevorrichtung in der Lage ist, das Gemisch in ein Individuum auszugeben.

29. Computerprogrammprodukt nach Anspruch 28, darüber hinaus einen fünften ausführbaren Abschnitt umfassend, um das Gemisch auszugeben, indem das Gemisch mit der Ausgabevorrichtung mittels einer automatisierten Injektionsvorrichtung in ein Individuum injiziert wird.

30. Computerprogrammprodukt nach Anspruch 28, darüber hinaus einen sechsten ausführbaren Abschnitt umfassend, um eine Dosierungsinformation aufzunehmen, welche einer ausgewählten Dosis des Gemisches, welche in das Individuum auszugeben ist, entspricht.

31. Computerprogrammprodukt nach Anspruch 30, darüber hinaus einen siebten ausführbaren Abschnitt umfassend, um das vorbestimmte Verhältnis vor dem zweiten ausführbaren Abschnitt zu bestimmen, welches auf der aufgenommenen Dosierungsinformation basiert.

32. Computerprogrammprodukt nach Anspruch 28, darüber hinaus einen achten ausführbaren Abschnitt umfassend, um ein Radioaktivitätsniveau des Gemisches vor dem dritten ausführbaren Abschnitt zu bestimmen, so dass das Gemisch derart an das Individuum ausgegeben werden kann, dass das Individuum einer ausgewählten Strahlungsdosis ausgesetzt ist.

## Revendications

1. Système pour administrer un mélange dans un individu, le système comportant :
au moins un conteneur, adapté pour contenir une ou plusieurs substances,
au moins un dispositif de distribution,
au moins un dispositif de mélange pour venir en contact avec le ou les conteneurs de manière à réduire la manipulation d'une ou plusieurs premières substances contenues dans ceux-ci et à mélanger au moins une partie de la ou des premières substances avec au moins une partie d'une ou plusieurs secondes substances en fonction d'un rapport prédéterminé pour former un mélange, le au moins un dispositif de mélange étant capable de guider le mélange jusqu'au moins un dispositif de distribution pour une administration de celui-ci dans l'individu,
un dispositif informatique en contact de manière opérationnelle avec le au moins un dispositif de mélange et capable de déterminer le rapport prédéterminé et coopérant avec le au moins un dispositif de mélange pour former le mélange en fonction du rapport prédéterminé, et
un premier dispositif de mesure de rayonnement en contact de manière opérationnelle avec le au moins un conteneur pour déterminer un niveau de radioactivité de la ou des premières substances ou de la ou des secondes substances et pour transmettre le niveau de radioactivité déterminé de la ou des premières substances ou de la ou des secondes substances au dispositif informatique de telle sorte que le rapport prédéterminé peut être sélectionné pour produire un mélange ayant un niveau de radioactivité sélectionné.

2. Système selon la revendication 1 dans lequel le au moins un dispositif de mélange comporte un ensemble de tubes stériles jetables pour mélanger au moins une partie de la ou des premières substances avec au moins une partie de la ou des secondes substances.

3. Système selon la revendication 1, dans lequel le au moins un conteneur comporte un premier dispositif de mémoire configuré pour être capable de recevoir et de mémoriser un ensemble de données contenant des données associées à la ou aux premières substances et dans lequel le au moins un dispositif de mélange est également capable d'interroger le premier dispositif de mémoire pour accéder à l'ensemble de données.

4. Système selon la revendication 1, comportant également un deuxième dispositif conteneur pour contenir la ou les secondes substances et pour pouvoir être en contact avec le au moins un dispositif de mélange pour délivrer la ou les secondes substances à celui-ci.

5. Système selon la revendication 1, dans lequel le au moins un dispositif de mélange est en outre capable d'être en contact avec le au moins un dispositif de distribution de manière à délivrer le mélange à celui-ci.

6. Système selon la revendication 1, dans lequel le au moins un dispositif de distribution comporte un dispositif d'injection adapté pour injecter au moins une partie du mélange dans l'individu.

7. Système selon la revendication 6, dans lequel le au moins un dispositif de distribution est capable d'être en contact de manière opérationnelle avec un dispositif d'injection automatique pour injecter au moins une partie du mélange dans l'individu.

8. Système selon la revendication 1, dans lequel le dispositif informatique est également capable de recevoir des informations de dosage, le dispositif informatique étant en outre capable de déterminer le rapport prédéterminé à partir des informations de dosage reçues par celui-ci.

9. Système selon la revendication 1, dans lequel le au moins un dispositif de distribution comporte un second dispositif de mesure de rayonnement en contact de manière opérationnelle avec celui-ci pour déterminer un niveau de radioactivité du mélange et pour transmettre le niveau de radioactivité déterminé du mélange au dispositif informatique de telle sorte que le au moins un dispositif de distribution peut être capable d'administrer une dose du mélange à l'individu correspondant à une dose de rayonnement sélectionnée.

10. Système selon la revendication 1, dans lequel le au moins un conteneur comporte également un premier dispositif de protection en contact de manière opérationnelle avec celui-ci pour protéger un utilisateur contre la ou les premières substances ou la ou les secondes substances contenues dans celui-ci.

11. Système selon la revendication 1, dans lequel le au moins un dispositif de distribution comporte en outre un second dispositif de protection en contact de manière opérationnelle avec celui-ci pour protéger un utilisateur contre le mélange contenu dans celui-ci.

12. Système selon la revendication 1, dans lequel le dispositif de distribution comporte une seringue.

13. Système pour administrer un mélange dans un individu, le système comportant :
un premier conteneur pour contenir une première substance,
un dispositif d'injection automatique capable d'être en contact avec le conteneur de manière à réduire la manipulation manuelle de la première substance contenue dans celui-ci et à mélanger au moins une partie de la première substance avec au moins une partie d'une seconde substance en fonction d'un rapport prédéterminé pour former un mélange, le dispositif d'injection automatique étant également capable d'injecter le mélange dans l'individu par l'intermédiaire d'un dispositif de distribution en contact de manière opérationnelle et sélective avec le dispositif d'injection automatique,
un dispositif informatique en contact de manière opérationnelle avec le dispositif d'injection automatique, le dispositif informatique étant capable de déterminer le rapport prédéterminé et configuré pour coopérer avec le dispositif d'injection automatique pour former le mélange en fonction du rapport prédéterminé, et
un premier dispositif de mesure de rayonnement en contact de manière opérationnelle avec le premier dispositif conteneur pour déterminer un niveau de radioactivité de la première substance ou de la seconde substance et pour transmettre le niveau de radioactivité déterminé de la première substance ou de la seconde substance au dispositif informatique de telle sorte que le rapport prédéterminé peut être sélectionné pour produire un mélange ayant un niveau de radioactivité sélectionné.

14. Système selon la revendication 13, dans lequel le dispositif d'injection automatique comporte un ensemble de tubes stériles jetables pour mélanger au moins une partie de la première substance avec au moins une partie de la seconde substance.

15. Système selon la revendication 13, dans lequel le premier dispositif conteneur comporte un premier dispositif de mémoire capable de recevoir et de mémoriser un ensemble de données contenant des données associées à la première substance et dans lequel le dispositif d'injection automatique est également capable d'interroger le premier dispositif de mémoire pour accéder à l'ensemble de données.

16. Système selon la revendication 13, dans lequel le dispositif de distribution est capable d'être en contact de manière opérationnelle avec le dispositif d'injection automatique de manière sensiblement étanche aux fluides pour réduire une manipulation manuelle du mélange contenu dans celui-ci.

17. Système selon la revendication 13, dans lequel le dispositif de distribution comporte une seringue.

18. Système selon la revendication 13, comportant également un troisième dispositif conteneur pour contenir la seconde substance et pour être en contact de manière sélective avec le dispositif d'injection automatique pour délivrer la seconde substance à celui-ci.

19. Système selon la revendication 13, dans lequel le dispositif informatique est également capable de recevoir des informations de dosage, le dispositif informatique étant également capable de déterminer le rapport prédéterminé à partir des informations de dosage reçues par celui-ci.

20. Système selon la revendication 13, dans lequel le dispositif de distribution comporte un second dispositif de mesure de rayonnement en contact de manière opérationnelle avec celui-ci pour déterminer un niveau de radioactivité du mélange et pour transmettre le niveau déterminé de radioactivité du mélange au dispositif informatique de telle sorte que le dispositif d'injection automatique peut être capable d'administrer une dose du mélange à l'individu correspondant à une dose de rayonnement sélectionnée.

21. Système selon la revendication 13, dans lequel le premier dispositif conteneur comporte également un premier dispositif de protection en contact de manière opérationnelle avec celui-ci pour protéger un utilisateur contre la première substance ou la seconde substance contenue dans celui-ci.

22. Système selon la revendication 13, dans lequel le dispositif de distribution comporte en outre un second dispositif de protection en contact de manière opérationnelle avec celui-ci pour protéger un utilisateur contre le mélange contenu dans celui-ci.

23. Procédé pour préparer un ou plusieurs mélanges pour une administration dans un individu, le procédé comportant les étapes consistant à :
mettre en contact de manière opérationnelle au moins un conteneur destiné à contenir une ou plusieurs premières substances, avec au moins un dispositif de mélange de manière à délivrer la ou les premières substances au dispositif de mélange et à réduire la manipulation de la ou des premières substances contenues dans le dispositif conteneur,
déterminer un niveau de radioactivité de la ou des premières substances ou d'une ou plusieurs secondes substances,
mélanger, en utilisant le dispositif de mélange, au moins une partie de la ou des premières substances et au moins une partie de la ou des secondes substances en fonction d'un rapport prédéterminé pour former le ou les mélanges ayant un niveau de radioactivité prédéterminé basé au moins en partie sur le niveau de radioactivité déterminé, et
guider le ou les mélanges jusqu'à un dispositif de distribution, le dispositif de distribution étant capable d'administrer le mélange dans l'individu.

24. Procédé selon la revendication 23, comportant également la mise en contact de manière opérationnelle du dispositif de distribution avec un dispositif d'injection automatique pour injecter automatiquement le mélange dans l'individu.

25. Procédé selon la revendication 23, comportant également la réception d'informations de dosage correspondant à une dose sélectionnée du mélange à administrer à l'individu.

26. Procédé selon la revendication 25, comportant en outre la détermination du rapport prédéterminé avant l'étape de mélange, sur la base des informations de dosage reçues.

27. Procédé selon la revendication 23, comportant de plus la détermination d'un niveau de radioactivité du mélange avant l'étape de guidage de telle sorte que le mélange peut être administré à l'individu de manière à exposer l'individu à une dose de rayonnement sélectionnée.

28. Produit de programme informatique capable de commander un dispositif de mélange pour former un mélange, le produit de programme informatique comportant un support de mémorisation lisible par ordinateur ayant des parties de code de programme lisibles par ordinateur mémorisées dans celui-ci, les parties de code de programme lisibles par ordinateur comportant :
une première partie exécutable pour mettre en contact de manière opérationnelle un dispositif conteneur destiné à contenir une première substance, avec le dispositif de mélange de manière à délivrer la première substance au dispositif de mélange,
une deuxième partie exécutable pour déterminer un niveau de radioactivité de la première substance ou d'une seconde substance,
une troisième partie exécutable pour commander le dispositif de mélange pour mélanger au moins une partie de la première substance et au moins une partie de la seconde substance en fonction d'un rapport prédéterminé pour former le mélange ayant un niveau de radioactivité prédéterminé basé au moins en partie sur le niveau de radioactivité déterminé, et
une quatrième partie exécutable pour guider le mélange jusqu'à un dispositif de distribution, le dispositif de distribution étant capable d'administrer le mélange dans un individu.

29. Produit de programme informatique selon la revendication 28, comportant également une cinquième partie exécutable pour administrer le mélange en injectant le mélange dans un individu avec le dispositif de distribution via un dispositif d'injection automatique.

30. Produit de programme informatique selon la revendication 28, comportant en outre une sixième partie exécutable pour recevoir des informations de dosage correspondant à une dose sélectionnée du mélange à administrer dans l'individu.

31. Produit de programme informatique selon la revendication 30, comportant de plus une septième partie exécutable pour déterminer le rapport prédéterminé avant la deuxième partie exécutable, sur la base des informations de dosage reçues.

32. Produit de programme informatique selon la revendication 28, comportant également une huitième partie exécutable pour déterminer un niveau de radioactivité du mélange avant la troisième partie exécutable de telle sorte que le mélange peut être administré à l'individu de manière à exposer l'individu à une dose de rayonnement sélectionnée.
